# EUROPÄISCHE PATENTSCHRIFT

(11) **EP 1 572 610 B1**
(45) Veröffentlichungstag und Bekanntmachung des Hinweises auf die Patenterteilung: **02.08.2006**
(21) Anmeldenummer: 03799469.6
(22) Anmeldetag: 03.12.2003
(51) Int. Cl.: C07C 29/17, C07C 29/149, C07C 31/20, C07C 67/347, C07C 69/593

(54) **VERFAHREN ZUR HERSTELLUNG VON 1,6-HEXANDIOL**
METHOD FOR THE PRODUCTION OF 1.6-HEXANEDIOL
PROCEDE DE PRODUCTION DE 1,6-HEXANEDIOL

(30) Priorität: 13.12.2002 DE 10258316
(43) Veröffentlichungstag der Anmeldung: 14.09.2005
(73) Patentinhaber: BASF Aktiengesellschaft, 67056 Ludwigshafen (DE)
(72) Erfinder: FISCHER, Rolf-Hartmuth, 69121 Heidelberg (DE); KRUG, Thomas, 67550 Worms (DE); HAUNERT, Andrea, 68163 Mannheim (DE); RÖPER, Michael, 67157 Wachenheim (DE); SIRCH, Tilman, 67105 Schifferstadt (DE); STÜER, Wolfram, 68165 Mannheim (DE)
(86) Internationale Anmeldenummer: PCT/EP2003/013634
(87) Internationale Veröffentlichungsnummer: WO 2004/054948

(56) Entgegenhaltungen:
- EP-A- 0 475 386
- EP-A- 0 552 463
- PATENT ABSTRACTS OF JAPAN Bd. 1995, Nr. 02, 31. März 1995 (1995-03-31) & JP 6 329567 A (NIPPON SHOKUBAI CO LTD), 29. November 1994 (1994-11-29) in der Anmeldung erwähnt
- BROOKHART, MAURICE ET AL: "Catalytic tail-to-tail dimerization of methyl acrylate using rhodium(III) catalysts" JOURNAL OF THE AMERICAN CHEMICAL SOCIETY , 113(7), 2777-9 CODEN: JACSAT; ISSN: 0002-7863, 1991, XP002278173

## Beschreibung

Die vorliegende Anmeldung betrifft ein Verfahren zur Herstellung von 1,6-Hexandiol hoher Reinheit durch Dimerisierung von Acrylsäureestern in Gegenwart von RhodiumKatalysatoren, gegebenenfalls Abtrennung von nicht umgesetzten Acrylestern, Hydrierung der so erhaltenen Dimerisierungsausträge in Gegenwart von überwiegend Kupfer enthaltenden Katalysatoren und destillative Reinigung des erhaltenen 1,6-Hexandiols auf eine Reinheit von mindestens 99,5 %.

Es ist bekannt, 1,6-Hexandiol durch katalytische Hydrierung von C₆-Verbindungen wie Adipinsäure oder Adipinsäurediestern herzustellen. Die lineare Dimerisierung von Acrylsäureestern eröffnet einen alternativen Zugang zu Hexandiol auf Basis einer C3-Rohstoffquelle. So kann durch Oxidation von Propan oder Propen zu Acrylsäure und anschließende Veresterung mit Methanol die für die Dimerisierung benötigte Ausgangsverbindung Methylacrylat hergestellt werden.

Verfahren zur Herstellung von Hexendisäuredimethylestern ausgehend von Methylacrylat sind an sich bekannt. Zahlreiche Katalysatoren sind in der Literatur beschrieben worden. Als katalytisch aktiv erwiesen sich bisher die Übergangsmetalle Ru, Rh, Ni und Pd.

Für die Rhodium-haltigen Katalysatoren konnten ausgehend von RhCl₃ durch Variation der Liganden einige aktive und selektive Katalysatorsysteme entwickelt werden.

EP-A 475 368 beschreibt die Dimerisierung von Acrylsäureestern wie Methylacrylat in Gegenwart spezifischer kationischer Rhodiumverbindungen als Katalysatoren. Es werden Isomerengemische enthaltend E- und Z-2-Hexendisäurediester und E- und Z-3-Hexendisäurediester erhalten.

Es ist weiterhin aus JP-A 06 329567, Beispiel 1, bekannt, Methylacrylat bei 65 °C in Gegenwart von Palladiumchlorid, Eisenchlorid und Eisennitrat zu Hexendisäuredimethylestern umzusetzen (Acrylester-Umsatz 60 %). Die Dehydroadipinsäuredimethylester-Selektivität nach destillativer Aufarbeitung betrug 95 %. So erhaltener Diester wurde in Methanol gelöst und bei 250 °C in Gegenwart eines Kupferchromit-Katalysators 4 Stunden lang hydriert. Die 1,6-Hexandiol-Ausbeute betrug 97 % (bezogen auf Hexendisäuredimethylester) und die 1,6-Hexandiol-Selektivität demnach 92 % (bezogen auf Acrylsäuremethylester).

Der Anteil an nicht linearen Dimeren beträgt nach JP-A 6100496, Beispiel 11%.

Es bestand die Aufgabe, ein Verfahren zur Herstellung von 1,6-Hexandiol mit einer Reinheit von mindestens 99,5 % ausgehend von Acrylsäureestern zu entwickeln. Dabei sollte das Dimerisierungsprodukt ohne aufwendige Reinigung für die Hydrierung verwendet werden können und bei der Hydrierung hohe 1,6-Hexandiol-Ausbeuten erzielbar sein. Der Hydrieraustrag sollte sich destillativ ohne großen Aufwand zu 1,6-Hexandiol einer Reinheit von mindestens 99,5 % aufarbeiten lassen.

Diese Aufgabe wird gelöst in einem Verfahren zur Herstellung von 1,6-Hexandiol mit einer Reinheit von ≥ 99,5 Gew.-% durch katalytische Dimerisierung von Acrylsäureestern und katalytische Hydrierung der so erhaltenen Hexendisäurediester zu 1,6-Hexandiol, bei dem man
a) C₁- bis C₈-Acrylsäureester in Gegenwart mindestens einer Rhodiumverbindung zu Gemischen aus überwiegend 2- und 3-Hexendisäurediestern dimerisiert,
b) den erhaltenen Dimerisierungsaustrag in Gegenwart von chromfreien, als Hydrierkomponente überwiegend Kupfer enthaltenden Katalysatoren hydriert und
c) das so erhaltene Roh-1,6-Hexandiol durch fraktionierende Destillation reinigt.

Gegenüber der Dimerisierung mit Palladium (Pd)-Katalysatoren (JP-A 06 329567) besitzt die Dimerisierung mit Rhodium (Rh)-Katalysatoren den Vorteil, dass höhere Acrylester-Umsätze und höhere Ausbeuten an linearen C6-Diestern erzielt werden. Der höhere Anteil an 2-Methyleno-glutarsäurediestern und Doppelbindungs-Isomeren dieser Verbindungen führt im Falle der Pd-katalysierten Dimerisierung nach Hydrierung zu einem höheren Anteil an 2-Methyl-1,5-pentandiol. Hiermit ist ein höherer Destillationsaufwand verbunden.

Die Acrylsäureester-Dimerisierung wird bevorzugt gemäß EP-A 475 386, auf deren Offenbarung hier ausdrücklich Bezug genommen wird, in Gegenwart von kationischen Rhodium-Verbindungen durchgeführt. Als kationische Rhodiumverbindung kommen insbesondere die in EP-A 475 386 offenbarten Verbindungen des Typs [L¹RhL²L³R]⁺X⁻ in Betracht, wobei
- L¹: ein anionischer Pentahapto-Ligand ist;
- L² und L³: für neutrale 2-Elektronen-Donorliganden stehen;
- R: ausgewählt wird aus der Gruppe, bestehend aus H, C₁-C₁₀-Alkyl, C₆-C₁₀₋Aryl- und C₇-C₁₀-Aralkyl-Liganden;
- X⁻: für ein nichtkoordinierendes Anion steht; und

und worin zwei oder drei von L², L³ und R gegebenenfalls verbunden sind. Anstelle von Rhodium können auch Ruthenium-Komplexverbindungen verwendet werden. Als Acrylsäureester kommen aliphatische und cycloaliphatische C₁- bis C₈-Ester wie zum Beispiel Acrylsäuremethylester C₁- bis C₈-Aryl- und Heteroarylester wie z.B. Acrylsäurephenylester in Frage. Die beiden Esterreste können dabei gleich oder verschieden sein. Bevorzugt werden als Arylsäureester Acrylsäurealkylester, insbesondere bevorzugt Methylacrylat, eingesetzt. Die Umsetzung wird diskontinuierlich oder kontinuierlich bei Temperaturen von -100 bis 150°C und Drucken von 0,1 bis 1 atm durchgeführt.

Das Reaktionsgemisch wird aufgearbeitet, indem die organischen Anteile ohne destillative Auftrennung der Einzelkomponenten als Gemisch vom Katalysator abgetrennt werden. War der Acrylsäureester-Umsatz nicht quantitativ, kann gegebenenfalls lediglich der unumgesetzte Acrylester abgetrennt und entweder in die Dimerisierungsstufe zurückgeführt oder anderweitig genutzt werden, wobei die Abtrennung und Rückführung in den Dimerisierungsschritt bevorzugt ist.

Der nach weitgehender Abtrennung von nicht umgesetztem Acrylsäureester erhaltene Dimerisierungsaustrag besteht neben 2-Methylen-glutarsäurediester, 2-Methyl-2-penten-disäurediester, Adipinsäurediester, Acrylester und Propionsäureester überwiegend aus E- und Z-2-Hexendisäurediester, E- und Z-3-Hexendisäurediester.

Der Dimerisierungsaustrag wird gegebenenfalls nach Abtrennung nicht umgesetzten Acrylesters, ohne weitere Reinigung der Hydrierung b) unterworfen.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind in H. Kropf, Houben-Weyl, Methoden der Organischen Chemie, Band IV/1c, Georg Thieme Verlag Stuttgart, 1980, S. 45 bis 67, und Beispiele für heterogene Katalysatoren sind in Houben-Weyl, Methoden der Organischen Chemie, Band IV/1 c, S. 16 bis 26, beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt, Nickel oder Rhenium enthalten.

Die Katalysatoren können allein aus den Aktivkomponenten bestehen oder die Aktivkomponenten können auf Trägem aufgebracht sein. Als Trägermaterialien eignen sich z.B. Cr₂O₃, Al₂O₃, SiO₂, ZrO₂, ZnO, BaO und MgO oder Mischungen daraus.

Insbesondere weist der Katalysator als katalytisch aktiven Hauptbestandteil Kupferoxid aus. Dieses ist auf einem oxidischen Träger aufgebracht. Ein geeignetes Trägermaterial ist Aluminiumoxid, dessen Verwendung gemäß einer Ausführungsform der vorliegenden Erfindung bevorzugt ist. Gemäß einer anderen Ausführungsform der vorliegenden Erfindung ist es bevorzugt, als Trägermaterial eine Kombination von Aluminiumoxid mit Zinkoxid im Gew.-Verhältnis 20 : 1 bis 1 : 20, vorzugsweise 5 : 1 bis 1 : 5 zu verwenden. Die Menge an Kupferoxid liegt bei Werten < 80 Gew.-%. Bevorzugte Katalysatorzusammensetzungen weisen < 70 Gew.-% Kupferoxid und > 30 Gew.-% Träger, besonders bevorzugte Katalysatoren 10 bis 65 Gew.-% Kupferoxid und 35 bis 90 Gew.-% Träger auf.

Weiterhin bevorzugt sind Hydrierkatalysatoren, wie sie in EP-A 552 463 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c ≥ 0, d > 0, a > b/2, b > a/4, a > c und a > d gilt und x die zur Wahrung der Elektroneutralität pro Formeleinheit erforderliche Anzahl von Sauerstoffionen bezeichnet. Die Herstellung dieser Katalysatoren kann beispielsweise nach den Angaben der EP-A 552 463 durch Fällung von schwerlöslichen Verbindungen aus Lösungen erfolgen, welche die entsprechenden Metallionen in Form ihrer Salze enthalten.

Im einzelnen kommen z. B. Katalysatoren der Zusammensetzungen von ungefähr 70 Gew.-% CuO, 20 Gew.% Al₂O₃ und 10 Gew.-% Mn₂O₃ in Betracht.

Optionsweise können die erfindungsgemäß verwendeten Hydrierkatalysatoren, die Crfrei sind, ein oder mehrere weitere Metalle oder eine Verbindung davon, vorzugsweise ein Oxid, aus den Gruppen 1 bis 14 (IA bis VIIIA und IB bis IVB der alten IUPAC-Nomenclatur) des Periodensystems der Elemente enthalten. Wird ein solches weiteres Oxid verwendet, wird vorzugsweise TiO₂, ZrO₂, SiO₂ und/oder MgO eingesetzt.

Die eingesetzten Katalysatoren können zudem ein Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel und Kupferpulver.

Die Katalysatoren lassen sich nach dem Fachmann bekannten Methoden herstellen. Bevorzugt sind Verfahren, bei denen das Kupferoxid fein verteilt und innig vermischt mit den anderen Bestandteilen anfällt, besonders bevorzugt sind Fällungsreaktionen. Dabei werden in einem Lösungsmittel gelöste Vorläuferverbindungen in Gegenwart weiterer löslicher oder im Lösungsmittel suspendierter Metallverbindungen mit einem Fällungsmittel ausgefällt, abfiltriert, gewaschen, getrocknet und gegebenenfalls calciniert. An dieser Stelle sei nochmals ausdrücklich auf die Offenbarung der EP-A 552 463 verwiesen.

Diese Ausgangsmaterialien können nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Alternativ können erfindungsgemäße Katalysatoren beispielsweise auch durch Aufbringen der Aktivkomponente auf einen Träger hergestellt werden, beispielsweise durch Tränken oder Aufdampfen. Weiterhin können erfindungsgemäße Katalysatoren durch Verformen einer heterogenen Mischung aus Aktivkomponente oder Vorläuferverbindung hiervon mit einer Trägerkomponente oder Vorläuferverbindung hiervon erhalten werden.

Bei der erfindungsgemäßen Hydrierung wird der Katalysator in reduzierter, aktivierter Form verwendet. Die Aktivierung erfolgt mit reduzierenden Gasen, vorzugsweise Wasserstoff oder Wasserstoff/Inertgas-Gemischen, entweder vor oder nach dem Einbau in den Reaktor, in dem das erfindungsgemäße Verfahren durchgeführt wird. Wurde der Katalysator in oxidischer Form in den Reaktor eingebaut, so kann die Aktivierung sowohl vor dem Anfahren der Anlage mit der erfindungsgemäßen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/Inertgas-Gemischen bei erhöhten Temperaturen, vorzugsweise zwischen 100 und 300°C. Bei der sogenannten in-situ-Aktivierung erfolgt die Aktivierung beim Hochfahren der Anlage durch Kontakt mit Wasserstoff bei erhöhter Temperatur.

Die Katalysatoren werden als Formkörper verwendet. Beispiele umfassen Stränge, Rippstränge, andere Extrudatformen, Tabletten, Ringe, Kugeln und Splitt.

Die BET-Oberfläche der Kupferkatalysatoren beträgt im oxidischen Zustand 10 bis 400 m²/g, vorzugsweise 15 bis 200 m²/g, insbesondere 20 bis 150 m²/g. Die Kupferoberfläche (N₂O-Zersetzung) des reduzierten Katalysators beträgt im Einbauzustand > 0,2 m²/g, vorzugsweise > 1 m²/g, insbesondere > 2 m²/g.

Die erfindungsgemäß verwendeten Katalysatoren besitzen im allgemeinen eine ausreichende Standzeit. Für den Fall, dass die Aktivität und/oder Selektivität des Katalysators dennoch im Laufe ihrer Betriebszeit sinken sollte, kann dieser durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, den Katalysator mit einem geeigneten Lösungsmittel, beispielsweise Methanol, THF oder gamma-Butyrolacton zu waschen und anschließend in einem Gasstrom zu trocknen.

Bevorzugt werden Heterogenkatalysatoren verwendet, die entweder fest angeordnet, als Wirbelbett oder als Suspension eingesetzt werden. Wird die Hydrierung in der Gasphase und über fest angeordnetem Katalysator durchgeführt, werden im allgemeinen Temperaturen von 150 bis 300°C bei Drücken von 1 bis 80 bar angewandt. Dabei wird mindestens so viel Wasserstoff als Hydriermittel und Trägergas verwendet, dass Edukte, Zwischenprodukte und Produkte während der Reaktion nie flüssig werden.

Erfolgt die Hydrierung in der Flüssigphase mit fest angeordnetem oder suspendiertem Katalysator, so wird sie im allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinander geschalteten Reaktoren durchgeführt werden. Die Hydrierung in Flüssigphase über einem Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden.

Die Hydrierung kann ohne oder mit Zusatz eines Lösungsmittels erfolgen. Als Lösungsmittel kommen Alkohole, Ether, Kohlenwasserstoffe, wie beispielsweise Methanol, i-Propanol, Ethanol, Dioxan, Tetrahydrofuran, n-Pentan in Frage. Dabei kommen 5 bis 70 %ige, bevorzugt 10 bis 60 %ige, besonders bevorzugt 15 bis 50 %ige Lösungen der Formylvaleriansäureester-Isomerengemische in Frage. Besonders bevorzugt ist, den Alkohol als Lösungsmittel zu verwenden, der auch bei der Hydrierung der Estergruppen freigesetzt wird.

Die Hydrierung kann diskontinuierlich, bevorzugt kontinuierlich erfolgen.

Die Katalysator-Belastung beträgt 0,01 bis 1, bevorzugt 0,05 bis 0,8, besonders bevorzugt 0,1 bis 0,5 kg zu hydrierende C₆-Diester/l Katalysator · Stunde.

Das Wasserstoff/Edukt-Molverhältnis ist ebenfalls ein Parameter, der einen wichtigen Einfluss auf die Wirtschaftlichkeit des erfindungsgemäßen Verfahrens hat. Aus wirtschaftlicher Sicht ist ein niedriges Wasserstoff/Edukt-Verhältnis wünschenswert. Die Untergrenze liegt bei einem Wert von 5, wobei jedoch generell höhere Wasserstoff/Edukt-Molverhältnisse von 20 bis 400 angewendet werden.

Um die erfindungsgemäß verwendeten Wasserstoff/Edukt-Molverhältnisse einzustellen, wird ein Teil des Wasserstoffs im Kreis gefahren. Hierzu setzt man im allgemeinen die dem Fachmann bekannten Kreisgasverdichter ein. Die chemisch durch die Hydrierung verbrauchte Wasserstoffmenge wird ergänzt. In einer bevorzugten Ausführungsform wird ein Teil des Kreisgases ausgeschleust, um Inertverbindungen zu entfemen. Der im Kreis geführte Wasserstoff kann auch, gegebenenfalls nach Vorheizen, zum Verdampfen des Eduktstroms benutzt werden.

Gemeinsam mit dem Wasserstoff-Kreisgas werden alle Produkte im Kreis geführt, die beim Kühlen des aus dem Hydrierreaktor austretenden Gasstroms nicht oder nicht vollständig auskondensieren. Die Kühltemperatur beträgt 0 bis 60°C, vorzugsweise 20 bis 45°C.

Der Umsatz, bezogen auf die Summe der 1,6-Hexandiol bildenden C₆-Diester beträgt über 95 %, insbesondere über 98 %.

Der Hydrieraustrag besteht im wesentlichen aus 1,6-Hexandiol und dem der Estergruppe entsprechenden Alkohol. Ein weiterer wichtiger Bestandteil ist 2-Methyl-1,5-pentandiol.

Der Hydrieraustrag wird durch fraktionierte Destillation in einer oder mehreren Kolonnen gereinigt.

Es war nicht vorauszusehen, dass die Hydrierung von nicht gereinigten Austrägen der Acrylester-Dimerisierung ein Roh-Hexandiol ergeben würde, das durch Destillation auf eine Reinheit von über 99,5 % gebracht werden kann. Insbesondere war nicht vorauszusehen, dass sich 2-Methyl-1,5-pentandiol als Isomeres des 1,6-Hexandiols mit vertretbarem Aufwand würde abtrennen lassen.

Das erfindungsgemäße Verfahren wird anhand der nachfolgenden Beispiele näher erläutert.

### Beispiel 1

### 1.1 Dimerisierung von Methylacrylat in Gegenwart vom Rh-Katalysator

Die Versuche wurden unter einer Atmosphäre aus getrocknetem und nachgereinigtem Argon mittels Standard-Schlenk Technik durchgeführt. Methylenchlorid wurde über P₂O₅ getrocknet, Methylacrylat (Firma Aldrich, stabilisiert mit Methoxyphenol) wurde über Molsieb 4 gelagert und ohne weitere Behandlung eingesetzt. Der Komplex Cp*Rh(C₂H₄)₂ (Cp* = Pentamethylcyclopentadienyl) wurde ausgehend von [Cp*Rh(Cl₂]₂ nach der Vorschrift von K. Moseley, J.W. Kang, P.M. Maitlis J. Chem. Soc. (A) 1970, 2875-2883 hergestellt. Das Ausgangsmaterial [Cp*Rh(Cl₂]₂ wurde nach der Vorschrift von B.L. Booth, R.N. Haszeldine, M. Hill J. Chem. Soc. (A) 1969, 1299-1303 synthetisiert. Die zur Aktivierung des Katalysators benötigte Säure HBAr^{F}₄ wurde nach M. Brookhart, B. Grant, A. F. Volpe Organometallics 1992, 11, 3920-3922 hergestellt. Dabei bezeichnet HBAr^{F}₄ das bis-Etherat der Tetrakis[3,5-bis(trifluormethyl)phenyl]-Borsäure.

Die Analyse der Reaktionsausträge erfolgte mittels GC (Gerät: Hewlett Packard 5820; Säule: HP-5; Länge: 30 m; Durchmesser: 0,25 mm; Filmdicke: 1.0 µ), wobei die Strukturaufklärung der Produkte zuvor mittels GC/MS-Kopplung erfolgte. Alle Angaben in Flächenprozent.

Analog zu Beispiel 14 in EP-A 475 386 wurden 20 mg (0.06 mmol) Cp*Rh(C₂H₄)₂ in einem geeigneten Reaktionsgefäß zunächst mit 80 ml Methylacrylat und anschließend bei 0°C mit einer Lösung der stöchiometrischen Menge (bezogen auf Rh) der Säure HBAr^{F}₄ in 10 ml CH₂Cl₂ versetzt. Die Mischung wurde auf 55°C erwärmt und für 4 h unter 1 bar Wasserstoff gerührt.

Daraufhin wurde der Reaktionsaustrag fraktioniert destilliert, um den homogen gelösten Rhodium-Katalysator abzutrennen. Dabei wurde bei Normaldruck zunächst ein Vorlauf von Leichtsiedem (Methylacrylat, Methylpropionat und Methylenchlorid) abgetrennt.

Als Hauptfraktion, die für die nachfolgende Hydrierung zum Einsatz kommt, wurde ein Gemisch der folgenden Zusammensetzung erhalten. Dabei wurden die verschiedenen isomeren 2-Methylen-glutarsäuredimethylester und Hexendisäuredimethylester jeweils zusammengefasst:

| | |
|---|---|
| Methylacrylat: | 0,15 % |
| Methylpropionat: | 0,10 % |
| 2-Methylen-glutarsäuredimethylester und Isomere: | 0,52 % |
| Hexendisäuredimethylester: | 98,72 % |
| Dimethyladipat: | 0,35 % |

Im Sumpf der Destillation blieben lediglich geringe Mengen Triester und Polymer zurück.

### 1.2 Hydrierung des nach 1.1 erhaltenen Dimerisierungsaustrags

In einem diskontinuierlichen Autoklavenversuch wurde ein Gemisch aus 30 g des in 1.1 charakterisierten Ester-Gemischs und 70 g Methanol in Gegenwart von 20 g Kupferkatalysator (60 % CuO, 30 % Al₂O₃, 10 % Mn₂O₃) 6 Stunden lang bei 210°C / 240 bar gerührt. Die Aktivierung des oxidischen Katalysators erfolgte vor der Hydrierung mit Wasserstoff bei 200°C/300 bar.

Laut gaschromatographischer Analyse (innerer Standard: Diethylenglykoldimethylether) betrug die 1,6-Hexandiol-Ausbeute 99 % (bezogen auf die nach 1.1 enthaltenen linearen C₆-Diester). Der rohe Hydrieraustrag enthielt 0,4 % 2-Methyl-1,5-pentandiol.

### 1.3 Destillation des Hydrieraustrags

Die Destillation von Hydrieraustrag, aus dem Methanol weitgehend entfernt war, an einer Drehbandkolonne (max. 160°C/1 mbar) ergab zwei Hauptfraktionen mit einer 1,6-Hexandiolreinheit von 99,6 %. 2-Methyl-1,5-pentandiol war nur mit 1000 ppm bzw. 300 ppm enthalten.

### Vergleichsbeispiel 1

### a) Dimerisierung gemäß JP-A 06 329 567 Beispiel 1

0,34 g (2 mmol) PdCl₂, 8,1 g (50 mmol) FeCl₃, 86 g (1 mmol) Methylacrylat und 2,0 g (5 mmol) Fe(NO₃)₃ · 9 H₂O wurden bei 65°C 15 h umgesetzt. Aus dem erhaltenen Reaktionsaustrag wurden die nach dem Abkühlen in fester Form vorliegenden Katalysatoranteile abfiltriert. Die organische Phase wurde durch Kugelrohrdestillation aufgearbeitet. Destilliert wurde erst bei 80°C/150 mbar, dann bei 50 mbar und zuletzt bei 1 mbar. Erhalten wurden 35,9 g nicht umgesetzter Acrylsäuremethylester und 12,4 g eines Destillatgemisches aus linearen und verzweigten ungesättigten C₆-Dicarbonsäuredimethylestern. Die 12,5 g Destillatgemische bestanden zu 84 Gew.-% aus linearen 2- und 3-Hexendisäuredimethylestern und zu 2,4 Gew.-% aus 2-Methylenglutarsäurediemethylester und Doppelbindungs-Isomeren dieser Verbindung. Das gaschromatographisch ermittelte Verhältnis von linearen zu verzweigten ungesättigten Estern betrug demnach 35 : 1. Als Destillationsrückstand fielen 9 g Feststoff an.

### b) Hydrierung des Gemisches aus linearen und verzweigten C₆₋Dicarbonsäuredimethyl-estern.

12 g des nach a) erhaltenen Gemisches aus linearen und verzweigten C₆₋Dicarbonsäure-diestem wurden in 100 g Methanol gelöst und in Gegenwart von 10 g des im erfindungsgemäßen Beispiel 1.2 vorstehend beschriebenen KupferKatalysators, wie dort beschrieben, hydriert.

Nach der Hydrierung wurde festgestellt, dass der Kupfer-Katalysator sich rot gefärbt hatte und dass die verwendeten 3 x 3 mm Tabletten vollständig zerfallen waren. Die gaschromatographische Analyse des Hydrieraustrags ergab, dass ganz überwiegend Adipinsäuredimethylester entstanden war. Eine Wiederholung des Versuchs führt zum gleichen Ergebnis.

Die Ergebnisse des Vergleichsbeispiels zeigen, dass die Pd-katalysierte Dimerisierung mit niedrigeren Acrylester-Umsätzen und einem niedrigeren Verhältnis von linearen zu verzweigten C₆-Dicarbonsäureestern verläuft:

### Verhältnis lineare zu verzweigte C₆-Dicarbonsäuredimethylester

| | |
|---|---|
| Rh-Dimerisierung: | 190 : 1 |
| Pd-Dimerisierung: | 35 : 1 |

Da die ungesättigten, verzweigten C₆-Dicarbonsäuredimethylester bei der Hydrierung 2-Methyl-1,5-pentandiol ergeben, das vom 1,6-Hexandiol abgetrennt werden muss, ist für die 1,6-Hexandiol-Reinigung, ausgehend von Pd-dimerisierten Roh-C₆₋Dicarbonsäuredimethylestern, ein höherer Destillationsaufwand notwendig. Der Vergleichsversuch zeigt auch, dass die Kupfer-Hydrierkata-lysatoren bei Verwendung der Diester aus der Pd-katalysierten Dimerisierung von Acrylester zerfallen und dass sie die Fähigkeit zur Hydrierung von Estergruppen verlieren.

## Patentansprüche

1. Verfahren zur Herstellung von 1,6-Hexandiol mit einer Reinheit von ≥ 99,5 Gew.-% durch katalytische Dimerisierung von Acrylsäureestern und katalytische Hydrierung der so erhaltenen Hexendisäurediester zu 1,6-Hexandiol, bei dem man
a) C₁- bis C₈-Acrylsäureester in Gegenwart mindestens einer Rhodiumverbindungen zu Gemischen aus überwiegend 2- und 3-Hexendisäurediestern dimerisiert,
b) den erhaltenen Dimerisierungsaustrag in Gegenwart von chromfreien, als Hydrierkomponente überwiegend Kupfer enthaltenden Katalysatoren hydriert und
c) das so erhaltene Roh-1,6-Hexandiol durch fraktionierende Destillation reinigt.

2. Verfahren nach Anspruch 1, **dadurch gekennzeichnet, dass** aus dem Dimerisierungsgemisch vor der Hydrierung nicht umgesetzter Acrylsäureester abgetrennt wird.

3. Verfahren nach einem der Ansprüche 1 oder 2, **dadurch gekennzeichnet, dass** als Acrylsäureester Methylacrylat eingesetzt wird.

4. Verfahren nach einem der Ansprüche 1 bis 3, **dadurch gekennzeichnet, dass** die Hydrierung an einem Katalysator, der in der oxidischen Form die Zusammensetzung
CuₐAl_{b}Zr_{c}Mn_{d}Oₓ
besitzt, wobei a > 0, b > 0, c ≥ 0, d > 0, a > b/2, b > a/4, a > c und a > d gilt und x die zur Wahrung der Elektroneutralität pro Formeleinheit erforderliche Anzahl von Sauerstoffionen bezeichnet, durchgeführt wird.

5. Verfahren nach einem der Ansprüche 1 bis 4, **dadurch gekennzeichnet, dass** die Dimerisierung bei -100 bis 150°C und Drücken von 0,1 bis 1,0 bar (0,1 bis 1 atm) durchgeführt wird.

6. Verfahren nach einem der Ansprüche 1 bis 5, **dadurch gekennzeichnet, dass** die Hydrierung bei 100 bis 350°C und Drücken von 30 bis 350 bar durchgeführt wird.

## Claims

1. A process for preparing 1,6-hexanediol having a purity of ≥ 99.5% by weight by catalytically dimerizing acrylic esters and catalytically hydrogenating the hexenedioic diesters obtained in this way to 1,6-hexanediol by
a) dimerizing C₁- to C₈-acrylic esters in the presence of at least one rhodium compound to give mixtures of predominantly 2- and 3-hexenedioic diesters,
b) hydrogenating the resulting dimerizing effluent in the presence of chromium-free catalysts comprising predominantly copper as the hydrogenation component and
c) purifying the crude 1,6-hexanediol obtained in this way by fractional distillation.

2. The process according to claim 1, wherein unconverted acrylic ester is removed from the dimerization mixture before the hydrogenation.

3. The process according to either of claims 1 or 2, wherein the acrylic ester used is methyl acrylate.

4. The process according to any of claims 1 to 3, wherein the hydrogenation is carried out over a catalyst which in the oxidic form has the composition
CuₐAl_{b}Zr_{c}Mn_{d}Oₓ
where a > 0, b > 0, c ≥ 0, d > 0, a > b/2, b > a/4, a > c and a > d, and x is the number of oxygen ions required per formula unit to preserve electronic neutrality.

5. The process according to any of claims 1 to 4, wherein the dimerization is carried out at from -100 to 150°C and pressures of from 0.1 to 1.0 bar (from 0.1 to 1 atm).

6. The process according to any of claims 1 to 5, wherein the hydrogenation is carried out at from 100 to 350°C and pressures of from 30 to 350 bar.

## Revendications

1. Procédé de préparation de 1,6-hexanediol présentant une pureté de ≥99,5 % en poids par dimérisation catalytique d'esters d'acide acrylique et hydrogénation catalytique des diesters de diacide hexénoïque ainsi obtenu en 1,6-hexanediol, dans lequel
a) on dimérise des esters d'acide acrylique en C₁-C₈ en présence d'au moins un composé de rhodium pour former des mélanges à base principalement de diesters de diacide 2- et 3-hexénoïque,
b) on hydrogène l'effluent de dimérisation obtenu en présence de catalyseurs exempts de chrome, qui contiennent principalement du cuivre comme composant d'hydrogénation, et
c) on purifie le 1,6-hexanediol brut ainsi obtenu par distillation fractionnée.

2. Procédé suivant la revendication 1, **caractérisé en ce que**, à partir du mélange de dimérisation, on isole des esters d'acide acrylique n'ayant pas réagi, avant l'hydrogénation.

3. Procédé suivant l'une des revendications 1 et 2, **caractérisé en ce que**, comme ester d'acide acrylique, on met en oeuvre de l'acrylate de méthyle.

4. Procédé suivant l'une des revendications 1 à 3, **caractérisé en ce que** l'hydrogénation est effectuée sur un catalyseur qui possède, dans la forme oxydée, la composition
CuₐAl_{b}Zr_{c}Mn_{d}Oₓ
dans laquelle a>0, b>0, c≥0, d>0, a>b/2, b>a/4, a>c et a>d et x représente le nombre d'ions oxygène nécessaire par unité de formule pour respecter l'électroneutralité.

5. Procédé suivant l'une des revendications 1 à 4, **caractérisé en ce que** la dimérisation est effectuée à -100 jusqu'à 150°C et à des pressions de 0,1 à 1,0 bar (0,1 à 1 atm).

6. Procédé suivant l'une des revendications 1 à 5, **caractérisé en ce que** l'hydrogénation est effectuée à 100 jusqu'à 350°C et à des pressions de 30 à 350 bars.
